(19) Europäisches Patentamt
European Patent Office
Office européen des brevets

(11) Veröffentlichungsnummer : **0 082 461**
**B1**

(12) # EUROPÄISCHE PATENTSCHRIFT

(45) Veröffentlichungstag der Patentschrift : **04.03.87**

(21) Anmeldenummer : **82111565.6**

(22) Anmeldetag : **14.12.82**

(51) Int. Cl.⁴ : **C 07 C 93/06**, C 07 C 93/12, C 07 C103/38, C 07 C 79/34, A 61 K 31/13

(54) **Substituierte Phenoxyalkanolamine und Phenoxyalkanol-cycloalkylamine, Verfahren zu ihrer Herstellung, diese Verbindungen enthaltende pharmazeutische Zubereitungen und Zwischenprodukte.**

(30) Priorität : **23.12.81 DE 3151201**

(43) Veröffentlichungstag der Anmeldung : **29.06.83 Patentblatt 83/26**

(45) Bekanntmachung des Hinweises auf die Patenterteilung : **04.03.87 Patentblatt 87/10**

(84) Benannte Vertragsstaaten : **AT BE CH DE FR GB IT LI LU NL SE**

(56) Entgegenhaltungen :
**DE-A- 2 649 605**
**FR-A- 2 081 523**
**FR-A- 2 139 740**
**FR-A- 2 289 172**
**Die Akte enthält technische Angaben, die nach dem Eingang der Anmeldung eingereicht wurden und die nicht in dieser Patentschrift enthalten sind.**

(73) Patentinhaber : **Beiersdorf Aktiengesellschaft**
**Unnastrasse 48**
**D-2000 Hamburg 20 (DE)**

(72) Erfinder : **Cohnen, Erich, Dr. Dipl.-Chem.**
**Heilwigstrasse 25**
**D-2000 Hamburg 20 (DE)**

EP 0 082 461 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind neue substituierte Phenoxyalkanolamine und Phenoxyalkanol-cycloalkylamine der allgemeinen Formel I

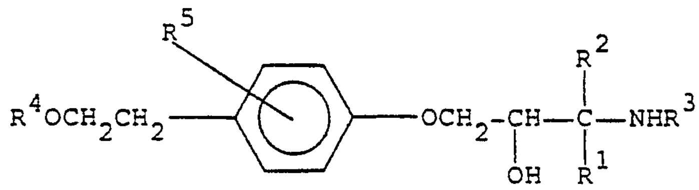

(I)

in der

$R^1$ und $R^2$, die gleich oder verschieden sein können, eine Alkylgruppe mit jeweils 1 bis 4 Kohlenstoffatomen oder

$R^1$ und $R^2$ zusammen die Gruppe $-(CH_2)_n-$ bedeuten,

worin

n die Zahl 4 oder 5 ist,

$R^3$ Wasserstoff, eine Alkylgruppe oder eine Acylgruppe mit jeweils 1 bis 6 Kohlenstoffatomen,

$R^4$ eine Alkylgruppe mit 1 bis 4 Kohlenstoffatomen oder die Cyclopropylmethylgruppe und

$R^5$ Wasserstoff, Halogen oder Alkyl mit 1 bis 6 Kohlenstoffatomen bedeutet

sowie deren Säureadditionssalze, Verfahren zu ihrer Herstellung und ihre Verwendung in pharmazeutischen Präparaten.

Ferner betrifft die Erfindung neue substituierte Phenoxynitroalkanole und Phenoxyhydroxyalkylnitrocycloalkane der allgemeinen Formel II

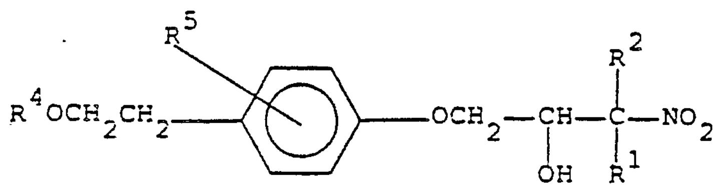

(II)

in der

$R^1$ und $R^2$, die gleich oder verschieden sein können, eine Alkylgruppe mit jeweils 1 bis 4 Kohlenstoffatomen oder

$R^1$ und $R^2$ zusammen die Gruppe $-(CH_2)_n-$ bedeuten,

worin

n die Zahl 4 oder 5 ist,

$R^4$ eine Alkylgruppe mit 1 bis 4 Kohlenstoffatomen oder die Cyclopropylmethylgruppe und

$R^5$ Wasserstoff, Halogen oder Alkyl mit 1 bis 6 Kohlenstoffatomen bedeutet, Verfahren zu ihrer Herstellung sowie ihre Verwendung als Zwischenprodukte für die Herstellung der erfindungsgemäßen Verbindungen der allgemeinen Formel I.

Die Zwischenprodukte der Formel II besitzen auch therapeutische Eigenschaften. Insbesondere können sie wie die erfindungsgemäßen Endprodukte der allgemeinen Formel I verwendet werden.

Die neuen Verbindungen der allgemeinen Formeln I und II enthalten ein oder zwei asymetrische Kohlenstoffatome. Daher sind auch die verschiedenen optischen Isomeren sowie die Diastereoisomeren Gegenstand der Erfindung ebenso wie die Additionssalze dieser Verbindungen mit Säuren. Racemate können nach an sich bekannten Methoden in ihre optischen Antipoden aufgetrennt werden, beispielsweise durch Verwendung optisch aktiver Säuren wie Weinsäure, Kampfersulfonsäure oder Dibenzoylweinsäure, oder als Ester oder Äther mit optisch aktiven Komponenten oder über Harnstoffeinschlußverbindungen.

Neue substituierte Phenoxyacetaldehyde der allgemeinen Formel III

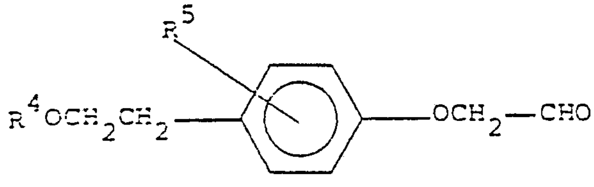

(III)

in der $R^4$ eine Alkylgruppe mit 1 bis 4 Kohlenstoffatomen oder die Cyclopropylmethylgruppe und $R^5$

Wasserstoff, Halogen oder Alkyl mit 1 bis 6 Kohlenstoffatomen bedeutet, finden Verwendung als Zwischenprodukte für die Herstellung der erfindungsgemäßen Verbindungen der allgemeinen Formeln I und II.

Aus der FR-A-2 081 523 und den deutschen Offenlegungsschriften 2 106 209 und 2 649 605 sind bereits Betarezeptoren blockierende Verbindungen bekannt, die aber nicht die erfindungsgemäßen Substituenten des α-Kohlenstoffatoms der Propoxy-Seitenkette und daher nicht die Vorteile der erfindungsgemäßen Verbindungen insbesondere bei der Glaukombehandlung besitzen.

Die hier gewählte Definition für Alkyle $R^1$, $R^2$, $R^3$, $R^4$ und $R^5$ und der Alkylreste der Acylgruppe $R^3$ bedeutet sowohl geradkettige als auch verzweigtkettige aliphatische, 1 bis 6 Kohlenstoffatome enthaltende Kohlenwasserstoffreste, wie zum Beispiel die Methylgruppe, Äthylgruppe, n-Propylgruppe, Isopropylgruppe, n-Butylgruppe, sek.-Butylgruppe, Isobutylgruppe, tert.-Butylgruppe, n-Pentylgruppe, Isopentylgruppe, Neopentylgruppe, Amylgruppe, n-Hexylgruppe, Isohexylgruppen und die verzweigten Hexylgruppen mit quartärem Kohlenstoffatom, wobei sich diese Definition der Alkylgruppen $R^1$, $R^2$ und $R^4$ auf Alkyle mit bis zu vier Kohlenstoffatomen bezieht. Besonders bevorzugt werden die Methylgruppe, die Äthylgruppe und die Propylgruppen.

$R^4$ ist vorzugsweise Methyl, Äthyl, Isopropyl oder Cyclopropylmethyl.

Bevorzugte Acylgruppen sind Alkanoylgruppen mit 1 bis 6 Kohlenstoffatomen, insbesondere die Formylgruppe, die Acetylgruppe und die Propionylgruppen.

Vorzugsweise befindet sich der Rest $R^5$ in der 2-Stellung der Phenylgruppe bzw. in ortho-Stellung zur 2-Hydroxy-3-amino-alkoxy-Seitenkette. Der Halogenrest $R^5$ ist Fluor, Chlor, Brom oder Jod, insbesondere aber Chlor oder Brom. Die bevorzugten Alkylreste $R^5$ sind Methyl und Äthyl.

$R^5$ ist vorzugsweise auch Wasserstoff.

Es werden erfindungsgemäße Verbindungen der Formeln I und II bevorzugt, die, mit der Definition, das $R^1$ und $R^2$ zusammen die Alkyliden-Gruppen $—(CH_2)_n—$ bedeuten, worin n 4 oder 5 ist, einen Cyclopentanring oder Cyclohexanring enthalten. Sie werden mit dieser Definition jeweils unter Einbeziehung des α-Kohlenstoffatoms der Propoxy-Seitenkette gebildet.

Weiterhin werden Verbindungen der Formeln I und II bevorzugt, in denen $R^1$ und $R^2$ Alkyl, insbesondere Methyl bedeuten und auch Verbindungen, in denen $R^1$ und $R^2$ Alkyl-, insbesondere Methylgruppen sind und $R^4$ Methyl oder Cyclopropylmethyl ist.

Die erfindungsgemäßen neuen Verbindungen der allgemeinen Formel I und ihre Säureadditionssalze besitzen wertvolle therapeutische Eigenschaften. Sie zeichnen sich durch eine cardioselektive $\beta_1$-adrenolytische Wirkung und blutdrucksenkende Eigenschaften aus und sind insbesondere zur Behandlung von Angina pectoris, Hypertonie und Herzarrhythmien geeignet. Weiterhin sind sie zur Glaukombehandlung geeignet, da sie den Augeninnendruck senken.

Die überraschend hohe Cardioselektivität der neuen Verbindungen ist unter anderem von der Di-Substitution des α-Kohlenstoffatoms der Propoxy-Kette abhängig. Als besonders wertvoll haben sich dabei Verbindungen herausgestellt, bei denen $R^3$ ein Wasserstoffatom bedeutet. Vorzugsweise sind dann $R^1$ und $R^2$ Alkylgruppen wie angegeben, insbesondere Methylgruppen.

Die folgenden Verbindungen der allgemeinen Formel I und deren Salze mit hohem therapeutischen Effekt werden besonders bevorzugt, und zwar in der Form der Racemate sowie in der Form optisch aktiver Isomerer:

1-[4-(2-Methoxyäthyl)-phenoxy]-3-amino-3-methyl-butan-2-ol
1-[4-(2-Cyclopropylmethoxyäthyl)-phenoxy]-3-amino-3-methyl-butan-2-ol

Die Verbindungen der vorliegenden Erfindung können beim Menschen oral oder parenteral in einer Dosierung von 20 mg bis 1 g, vorzugsweise 50 mg bis 500 mg, pro Tag angewendet werden, insbesondere in unterteilten Dosen, zum Beispiel zwei- oder dreimal täglich. Eine Dosis von 1 bis 10 mg/kg i. v. senkt bei der Katze die isoprenalinbedingte Tachykardie um 25 % ($ED_{25}$). Für 1-[4-(2-Methoxyäthyl)-phenoxy]-3-amino-3-methyl-butan-2-ol beträgt diese Dosis 1,75 mg.

Die Tagesdosis ist individuell abzustimmen, weil sie von der Rezeptorenempfindlichkeit und dem Sympathikotonus des Patienten abhängt. Zweckmäßigerweise wird die Behandlung mit niederen Dosen begonnen und dann gesteigert.

Zur Hypertoniebehandlung werden bevorzugt Dosen von 50 bis 200 mg, verteilt auf zwei oder drei Gaben, täglich verabreicht. Die Behandlung der Angina pectoris erfolgt vorzugsweise mit 50 bis 200 mg pro Tag, verteilt auf zwei Gaben. Zur Bekämpfung von Herzrhythmusstörungen werden 200 bis 400 mg täglich, verteilt auf zwei bis drei Gaben, verabreicht.

Zur Behandlung des erhöhten Augeninnendrucks werden 0,1 bis 4 %ige wäßrige isotone Lösungen in Dosierungen von einem oder mehreren Tropfen ein oder mehrmals täglich lokal am Auge angewendet.

Gemäß der Erfindung werden pharmazeutische Zusammensetzungen geschaffen, die eine Verbindung der Formel I oder deren pharmazeutisch verträgliche Salze, zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups,

Suspensionen und Flüssigkeiten, oder parenteral in Form von Lösungen oder Suspensionen verabreicht werden. Oral zu verabreichende Präparate können ein oder mehrere Zusätze, wie Süßungsmittel, Aromatisierungsmittel, Farbstoffe und Konservierungsmittel, enthalten. Tabletten können den Wirkstoff mit üblichen, pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Äthanol, Propylenglycol, Äther des Tetrahydrofurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyäthylenstearat und Polyoxyäthylensorbitanmonooleat, und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90 % insbesondere 1 bis 90 %, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in einer Menge von 50-100 mg.

Die Verbindungen der allgemeinen Formel I sind nach folgenden Verfahren erhältlich :

Das Verfahren zur Herstellung der Phenoxyalkanolamine und Phenoxyalkanol-cycloalkylamine gemäß der allgemeinen Formel I, mit der oben angegebenen Bedeutung der Reste $R^1$ bis $R^5$, ist dadurch gekennzeichnet, daß man Verbindungen der allgemeinen Formel II

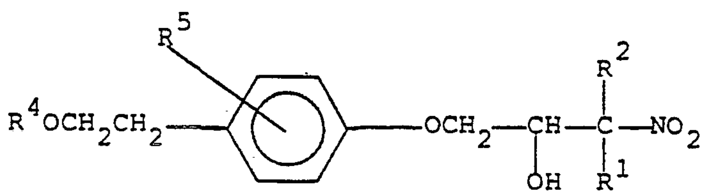

(II)

mit der oben angegebenen Bedeutung von $R^1$, $R^2$, $R^4$ und $R^5$ reduziert und die entstandene Aminogruppe gegebenenfalls alkyliert oder acyliert.

Die Reduktion erfolgt entweder mit Palladium/Wasserstoff in alkoholischer Lösung in Gegenwart von Eisessig bei 20 bis 100 °C und 5 bis 10 Bar oder mit Zink/Salzsäure bei Temperaturen zwischen 50 und 70 °C.

Die Alkylierung der Aminogruppe erfolgt entweder mit einem Alkylierungsmittel wie zum Beispiel Dialkylsulfat oder Tosylalkylester oder durch Reaktion mit einem Aldehyd und nachfolgende Reduktion des Azomethins mit einem Borhydrid.

Die Einführung der Methylgruppe ($R^3$=$CH_3$) wird am zweckmäßigsten durch Formylierung der Aminogruppe mit Ameisensäure/Acetanhydrid und anschließende Reduktion des Formamids mit Lithiumaluminiumhydrid vorgenommen.

Die Einführung der Acylgruppen wird mit entsprechenden Carbonsäureanhydriden oder Carbonsäurechloriden in Gegenwart eines basischen Katalysators oder auch durch Erhitzen der Komponenten durchgeführt. Die gegebenenfalls gebildeten Acylester werden durch alkalische Hydrolyse gespalten.

Das Verfahren zur Herstellung der Phenoxynitroalkanole und Phenoxyhydroxyalkyl-nitrocycloalkane gemäß der allgemeinen Formel II, mit der oben angegebenen Bedeutung der Reste $R^1$, $R^2$, $R^4$ und $R^5$, ist dadurch gekennzeichnet, daß man Verbindungen der allgemeinen Formel III

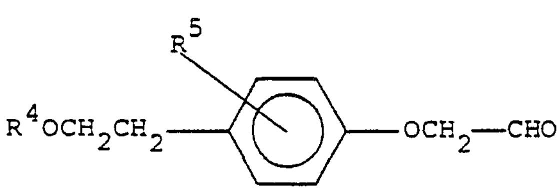

(III)

mit der oben angegebenen Bedeutung von $R^4$ und $R^5$, mit Verbindungen der allgemeinen Formel IV

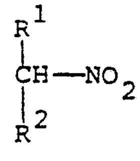

(IV)

mit der oben angegebenen Bedeutung von $R^1$ und $R^2$, umsetzt.

Die Nitroalkane bzw. Nitrocyclopentan und Nitrocyclohexan der Formel IV werden mit dem Aldehyd der Formel III in alkoholischer Lösung in Gegenwart von Natriumalkoholat bei Raumtemperatur kondensiert.

Die Nitroverbindungen der Formel IV sind bekannt oder nach bekannten Verfahren erhältlich.

Das Verfahren zur Herstellung der Aldehyde der allgemeinen Formel III mit der oben angegebenen Bedeutung von $R^4$ und $R^5$, ist dadurch gekennzeichnet, daß man Verbindungen der allgemeinen Formel V

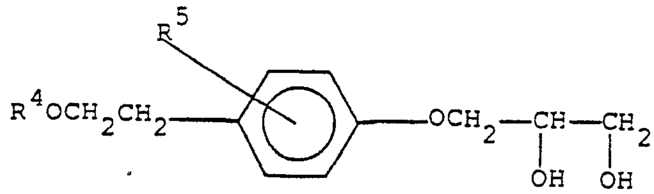

(V)

mit der angegebenen Bedeutung von $R^4$ und $R^5$, oxidativ spaltet.

Die Verbindungen der Formel III sind insbesondere zugänglich aus Verbindungen der allgemeinen Formel V durch oxidative Spaltung mit Blei (IV) acetat in Essigester, Benzol, Toluol oder Natriummetaperjodat in Alkohol/Wasser-Gemischen.

Die als Ausgangsmaterialien verwendeten Verbindungen der Formel V sind neue Verbindungen. Sie können aus den entsprechenden bekannten Phenolen durch Umsetzung mit 2,3-Epoxipropanol mit katalytischen Mengen eines quartären Amins in einem Lösungsmittel oder auch durch direktes Zusammenschmelzen der Reaktionspartner bei Temperaturen zwischen 120 und 160 °C hergestellt werden. Sie sind wertvolle Zwischenprodukte bei der Herstellung der erfindungsgemäßen Endprodukte.

Die Verbindungen der allgemeinen Formel I können entweder als Basen oder in der Form ihrer Salze aus den Reaktionsgemischen isoliert werden.

Sie lassen sich als Basen mit geeigneten anorganischen oder organischen Säuren nach bekannten Verfahren in Salze überführen. Bevorzugt werden physiologisch verträgliche Salze. Hierfür sind als anorganische Säuren beispielsweise Halogenwasserstoffsäuren, zum Beispiel Salzsäure oder Schwefelsäure und als organische Säuren zum Beispiel Fumarsäure und Maleinsäure geeignet. Zur Herstellung wird die heiße alkalische Lösung der Base mit der alkoholischen Lösung einer geeigneten Säure versetzt und man erhält nach Ätherzusatz das Salz.

Die folgenden Beispiele dienen zur Erläuterung der Erfindung :

## Beispiel 1

1-[4-(2-Methoxyäthyl)-phenoxy]-3-amino-3-methyl-butan-2-ol

19 g 1-[4-(2-Methoxyäthyl)-phenoxy]-3-nitro-3-methyl-butan-2-ol werden in einem Gemisch von 250 ml Ethanol und 50 ml Eisessig in Gegenwart von 4 g Palladium-Kohle (10 % Pd) bei 60 °C und 6 Bar hydriert. Nach Filtration und Abdampfen des Lösungsmittels wird der Rückstand mit 2 n NaOH alkalisiert und mit $CH_2Cl_2$ extrahiert. Man wäscht die organische Phase mit Wasser, trocknet mit $Na_2SO_4$ und erhält nach Eindampfen 15 g des Amins, das mit äthanolischer Salzsäure in das Hydrochlorid überführt wird.

Fp. 120-122 °C (Essigester).

Analog Beispiel 1 werden die in Tabelle 1 aufgeführten Verbindungen der allgemeinen Formel I dargestellt (in diesen Beispielen 2 bis 5a ist $R^5$ Wasserstoff) :

Tabelle 1

($R^5$=H)

| Beisp. Nr. | $R^1$ | $R^2$ | $R^3$ | $R^4$ | Fp. °C | Salz |
|---|---|---|---|---|---|---|
| 2 | $CH_3$ | $CH_3$ | H | $-CH_2$- 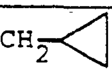 | 176-177 | Fumarat |
| 3 | $CH_3$ | $CH_3$ | H | $-CH_2CH_2CH_3$ | 174-176 | Fumarat |
| 3a | $CH_3$ | $CH_3$ | H | isopropyl | | Fumarat |
| 4 | $CH_3$ | $CH_2CH_3$ | H | $CH_3$ | 156-158 | Fumarat |
| 5 | $-(CH_2)_4-$ | | H | $CH_3$ | 182-183 | Fumarat |
| 5a | $-(CH_2)_5-$ | | H | $CH_3$ | 77-78 | Base |

Beispiel 6

1-[4-(2-Methoxyäthyl)-phenoxy]-3-amino-3-methyl-butan-2-ol

Zu 20 g 1-[4-(2-Methoxyäthyl)-phenoxy]-3-nitro-3-methyl-butan-2-ol in 400 ml Ethanol und 60 ml konzentrierter Salzsäure werden bei 50-60 °C nach und nach 18,3 g Zinkstaub gegeben. Man rührt eine Stunde bei dieser Temperatur, filtriert und dampft das Lösungsmittel ab. Nach Zugabe von 200 ml 40 %iger NaOH extrahiert man das Amin mit Methylenchlorid. Wie in Beispiel 1 beschrieben, erhält man das Hydrochlorid.

Fp. 119-120 °C.

Beispiel 6a

Analog Beispiel 6 wurde 1-[2-Chlor-4-(2-methoxyäthyl)-phenoxy]-3-amino-3-methyl-butan-2-ol erhalten.

Fp. 184-186 °C (Fumarat).

Beispiel 6b

Analog Beispiel 6 wurde 1-[2-Methyl-4-(2-methoxyäthyl)-phenoxy]-3-amino-3-methyl-butan-2-ol erhalten.

Beispiel 7

1-[4-(2-Methoxyäthyl)-phenoxy]-3-methylamino-3-methyl-butan-2-ol

Zu 5,1 g 1-[4-(2-Methoxyäthyl)-phenoxy]-3-amino-3-methyl-butan-2-ol in 50 ml Ameisensäure werden bei Raumtemperatur 15 ml Acetanhydrid getropft. Nach 15 Stunden werden 10 ml Wasser zugesetzt und es wird zur Trockne eingedampft. Nach üblicher Aufarbeitung erhält man 4,5 g der N-Formylverbindung als Öl, das in 50 ml THF mit 2 g $LiAlH_4$ reduziert wird. Man erhält 3,3 g der Base, die mit äthanolischer HCl in das Hydrochlorid überführt wird.

Fp. 86-88 °C.

Beispiel 8

1-[4-(2-Methoxyäthyl)-phenoxy]-3-hexylamino-3-methyl-butan-2-ol

5,1 g 1-[4-(2-Methoxyäthyl)-phenoxy]-3-amino-3-methyl-butan-2-ol werden mit 2,2 g Capronaldehyd in 50 ml Toluol unter Abdestillation des Wassers zum Azomethin umgesetzt. Nach Entfernen des Toluols nimmt man den Rückstand in 50 ml Methanol auf und reduziert bei 10-15 °C mit 1,9 g $NaBH_4$.

Nach üblicher Aufarbeitung und Reinigung durch Säulenchromatographie an Kieselgel erhält man 5 g der Base, die mit äthanolischer HCl und nach Kristallisation mit Essigester/Ether 3,9 g Hydrochlorid ergibt.

Fp. 135-137 °C.

Beispiel 9

1-[4-(2-Methoxyäthyl)-phenoxy]-3-isopropylamino-3-methyl-butan-2-ol

5,06 g (0,02 mol) 1-[4-(2-Methoxyäthyl)-phenoxy]-3-amino-3-methyl-butan-2-ol werden mit 4,3 g (0,02 mol) Toluol-4-sulfonsäure-isopropyl-ester zwei Stunden auf 140 °C erhitzt. Nach Zugabe von 100 ml Toluol und 100 ml 2 N NaOH wird eine Stunde gerührt, die Toluolphase abgetrennt, mit Wasser geschüttelt und im Vakuum eingedampft. Nach Säulenchromatographie an Kieselgel erhält man 3,5 g der obengenannten Verbindung, die mit äthanolischer HCl in das Hydrochlorid überführt wird.

Beispiel 10

1-[4-(2-Methoxyäthyl)-phenoxy]-3-acetamido-3-methyl-butan-2-ol

5,7 g 1-[4-(2-Methoxyäthyl)-phenoxy]-3-amino-3-methyl-butan-2-ol werden mit 30 ml Acetanhydrid 2

Stunden bei Raumtemperatur gerührt und nach Entfernen des überschüssigen Anhydrids wird der Rückstand 15 Stunden in 50 ml 1 N methanolischer Natronlauge stehengelassen. Nach üblicher Aufarbeitung erhält man 5,1 g der Acetamido-Verbindung.

Beispiel 11

1-[4-(2-Methoxyäthyl)-phenoxy]-3-nitro-3-methyl-butan-2-ol

Zu einer Lösung von 2 g Natrium in 150 ml Methanol tropft man nacheinander 39,3 g 2-Nitropropan und 27 g 4-(2-Methoxyäthyl)-phenoxy-acetaldehyd. Nach 15 stündigem Rühren bei Raumtemperatur wird die Lösung auf Wasser gegossen und mit 2 n HCl angesäuert. Nach Extraktion mit $CHCl_3$ erhält man nach Kristallisation aus Diisopropyläther 23,3 g der im Titel genannten Nitroverbindung.

Fp. 92-94 °C.

Analog Beispiel 11 werden die in Tabelle 2 aufgeführten Verbindungen der allgemeinen Formel II hergestellt (in diesen Beispielen 12 bis 15 ist $R^5$ Wasserstoff) :

Tabelle 2

($R^5$=H)

| Beisp. Nr. | $R^1$ | $R^2$ | $R^4$ | Fp. °C |
|---|---|---|---|---|
| 12 | $CH_3$ | $CH_3$ | $-CH_2-$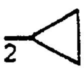 | 64 - 66 |
| 13 | $CH_3$ | $CH_3$ | $-CH_2CH_2CH_3$ | 82 - 84 |
| 14 | $CH_3$ | $CH_2CH_3$ | $CH_3$ | Öl |
| 15 | $-(CH_2)_4-$ | | $CH_3$ | Öl |

Beispiel 16

4-(2-Methoxy-äthyl)-phenoxy-acetaldehyd

39 g 4-(2-Methoxy-äthyl)-phenol werden nach Zusatz von 0,5 g Benzyl-tributylammoniumchlorid auf ca. 120 °C erhitzt und tropfenweise innerhalb von 15 Minuten mit 20,4 g 2,3-Epoxypropanol versetzt. Man läßt abkühlen und löst den Sirup in $CHCl_3$, wäscht mit Wasser und reinigt den $CHCl_3$-Rückstand durch präparative HPLC. Man erhält 50 g 3-[4-(2-Methoxyäthyl)-phenoxy]-propan-1,2-diol.

Fp. ca. 25 °C.

Zu 12 g obigen Diols in 100 ml Äthanol wird bei 10-15 °C eine Lösung von 2,5 g Natriummetaperjodat in 100 ml Wasser und 13 ml 0,1 n Borsäure zugetropft. Nach einer Stunde wird das $NaJO_3$ abfiltriert, das Filtrat mit Wasser verdünnt und der Aldehyd mit $CHCl_3$ extrahiert. Nach Abdampfen des Lösungsmittels verbleiben 11 g 4-(2-Methoxy-äthyl)-phenoxy-acetaldehyd als Mono-äthylacetal.

Fp. des 2,4-Dinitrophenylhydrazons : 106-108 °C.

Beispiel 17

Herstellung von Tabletten

Tabletten, welche die nachstehend angegebenen Bestandteile enthalten, werden nach bekannten Arbeitsweisen hergestellt. Diese sind für die Behandlung von Hypertonie in einer Dosierungsmenge von 50 mg zweimal täglich, für die Behandlung von Angina pectoris mit einer Menge von 50 mg zweimal täglich und zur Behandlung von Arrhythmien mit einer Menge von dreimal 100 mg täglich geeignet.

| | Tablette A | Tablette B |
|---|---|---|
| 1-[4-(2-Methoxyäthyl)-phenoxy]-3-amino-3-methyl-butan-2-ol | 50 mg | 100 mg |
| Lactose | 189 mg | 134 mg |
| Maisstärke | 25 mg | 25 mg |
| Magnesiumstearat | 1 mg | 1 mg |

Beispiel 18

Herstellung von Augentropfen

Augentropfen, die die folgenden Bestandteile enthalten, lassen sich in bekannter Weise herstellen. Sie können in einer Dosis von ein bis zwei Tropfen pro Auge ein bis zweimal täglich, vorzugsweise ein Tropfen zweimal täglich, verwendet werden :

| | |
|---|---|
| 1-[4-(2-Methoxyäthyl)-phenoxy]-3-amino-3-methyl-butan-2-ol | 0,5 g |
| isotone wäßrige Lösung (Ringerlösung) | ad 100 ml |

**Patentansprüche** (für die Vertragsstaaten : BE, CH, DE, FR, GB, IT, LI, LU, NL, SE)

1. Substituierte Phenoxyalkanolamine und Phenoxyalkanol-cycloalkylamine der allgemeinen Formel I

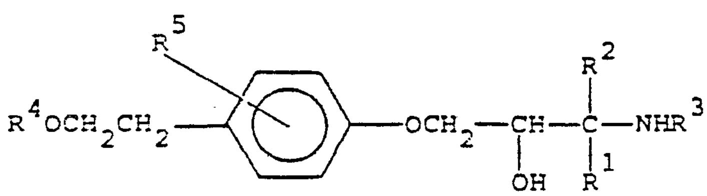

(I)

in der

$R^1$ und $R^2$, die gleich oder verschieden sein können, eine Alkylgruppe mit jeweils 1 bis 4 Kohlenstoffatomen oder

$R^1$ und $R^2$ zusammen die Gruppe $-(CH_2)_n-$ bedeuten,

worin

n die Zahl 4 oder 5 ist,

$R^3$ Wasserstoff, eine Alkylgruppe oder eine Acylgruppe mit jeweils 1 bis 6 Kohlenstoffatomen,

$R^4$ eine Alkylgruppe mit 1 bis 4 Kohlenstoffatomen oder die Cyclopropylmethylgruppe und

$R^5$ Wasserstoff, Halogen oder Alkyl mit 1 bis 6 Kohlenstoffatomen bedeutet,

sowie deren Säureadditionssalze.

2. Phenoxyalkanolamine und Phenoxyalkanol-cycloalkylamine nach Anspruch 1, gemäß der allgemeinen Formel I, in der $R^1$ und $R^2$ jeweils Methyl bedeuten.

3. Phenoxyalkanolamine und Phenoxyalkanol-cycloalkylamine nach Anspruch 1 oder 2, gemäß der allgemeinen Formel I, in der $R^1$ und $R^2$ jeweils Methyl bedeuten und $R^4$ Methyl oder Cyclopropylmethyl ist.

4. Phenoxyalkanolamine und Phenoxyalkanol-cycloalkylamine nach Anspruch 1, 2 oder 3, gemäß der allgemeinen Formel I, in der $R^3$ und/oder $R^5$ Wasserstoff bedeuten.

5. 1-[4-(2-Methoxyäthyl)-phenoxy]-3-amino-3-methyl-butan-2-ol.

6. 1-[4-(2-Cyclopropylmethoxyäthyl)-phenoxy]-3-amino-3-methyl-butan-2-ol.

7. Substituierte Phenoxynitroalkanole und Phenoxyhydroxyalkyl-nitrocycloalkane der allgemeinen Formel II

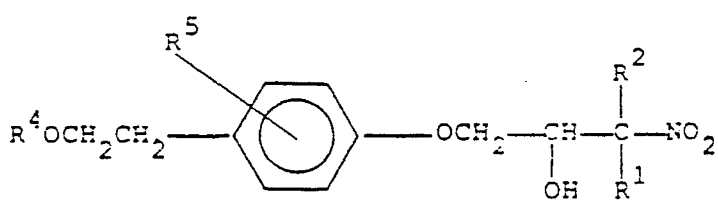

(II)

in der

$R^1$ und $R^2$, die gleich oder verschieden sein können, eine Alkylgruppe mit jeweils 1 bis 4 Kohlenstoffatomen oder

$R^1$ und $R^2$ zusammen die Gruppe $-(CH_2)_n-$ bedeuten,

worin

n die Zahl 4 oder 5 ist,

$R^4$ eine Alkylgruppe mit 1 bis 4 Kohlenstoffatomen oder die Cyclopropylmethylgruppe und

$R^5$ Wasserstoff, Halogen oder Alkyl mit 1 bis 6 Kohlenstoffatomen bedeutet.

8. Verfahren zur Herstellung der Phenoxyalkanolamine und Phenoxyalkanol-cycloalkylamine gemäß der allgemeinen Formel I, mit der oben angegebenen Bedeutung der Reste $R^1$ bis $R^5$, dadurch gekennzeichnet, daß man Verbindungen der allgemeinen Formel II

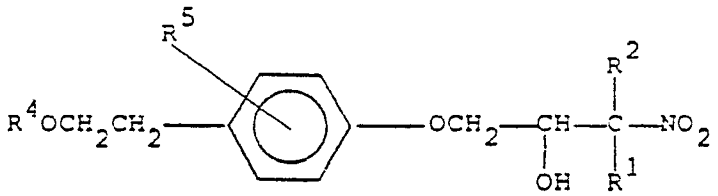

(II)

mit der oben angegebenen Bedeutung von $R^1$, $R^2$, $R^4$ und $R^5$, reduziert und die entstandene Aminogruppe gegebenenfalls alkyliert oder acyliert.

9. Verfahren zur Herstellung der Phenoxynitroalkanole und Phenoxyhydroxyalkyl-nitrocycloalkane gemäß der allgemeinen Formel II, mit der oben angegebenen Bedeutung der Reste $R^1$, $R^2$, $R^4$ und $R^5$, dadurch gekennzeichnet, daß man Verbindungen der allgemeinen Formel III

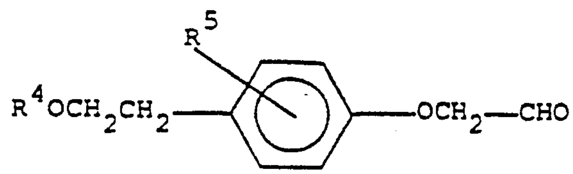

(III)

mit der oben angegebenen Bedeutung von $R^4$ und $R^5$, mit Verbindungen der allgemeinen Formel IV

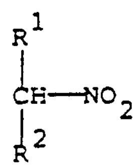

(IV)

mit der oben angegebenen Bedeutung von $R^1$ und $R^2$, umsetzt.

10. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

11. Verbindungen der allgemeinen Formel I oder deren physiologisch verträgliche Säureadditionssalze zur Verwendung als Arzneimittel.

12. Verbindungen der allgemeinen Formel I oder deren physiologisch verträgliche Salze zur Glaukombehandlung.

13. 1-[4-(2-n-Propoxyethyl)-phenoxy]-3-amino-3-methyl-butan-2-ol.

14. 1-[2-Chloro-4-(2-methoxyethyl)-phenoxy]-3-amino-3-methyl-butan-2-ol.

**Patentansprüche** (für den Vertragsstaat AT)

1. Verfahren zur Herstellung neuer substituierter Phenoxyalkanolamine und Phenoxyalkanol-cycloalkylamine der allgemeinen Formel I

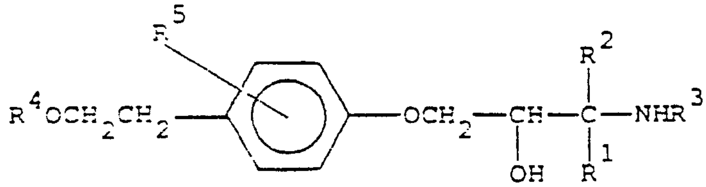

(I)

in der

$R^1$ und $R^2$, die gleich oder verschieden sein können, eine Alkylgruppe mit jeweils 1 bis 4 Kohlenstoffatomen oder

$R^1$ und $R^2$ zusammen die Gruppe $-(CH_2)_n-$ bedeuten.

worin

n die Zahl 4 oder 5 ist.

$R^3$ Wasserstoff, eine Alkylgruppe oder eine Acylgruppe mit jeweils 1 bis 6 Kohlenstoffatomen,

$R^4$ eine Alkylgruppe mit 1 bis 4 Kohlenstoffatomen oder die Cyclopropylmethylgruppe und

$R^5$ Wasserstoff, Halogen oder Alkyl mit 1 bis 6 Kohlenstoffatomen bedeutet,

sowie deren Säureadditionssalzen, dadurch gekennzeichnet, daß man Verbindungen der allgemeinen Formel II

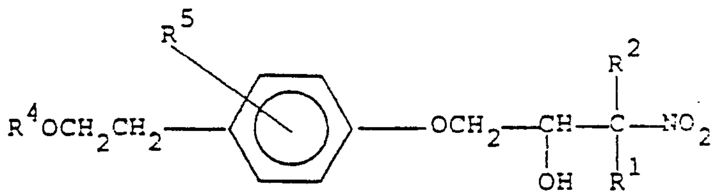

(II)

mit der oben angegebenen Bedeutung von $R^1$, $R^2$, $R^4$ und $R^5$, reduziert und die entstandene Aminogruppe gegebenenfalls alkyliert oder acyliert.

2. Verfahren nach Anspruch 1 zur Herstellung von 1-[4-(2-Methoxyäthyl)-phenoxy]-3-amino-3-methyl-butan-2-ol, dadurch gekennzeichnet, daß man 1-[4-(2-Methoxyäthyl)-phenoxy]-3-nitro-3-methyl-butan-2-ol zur Aminoverbindung der Formel I reduziert.

3. Verfahren nach Anspruch 1 zur Herstellung von 1-[4-(2-Cyclopropylmethoxyäthyl)-phenoxy]-3-amino-3-methyl-butan-2-ol, dadurch gekennzeichnet, daß man 1-[4-(2-Cyclopropylmethoxyäthyl)-phenoxy]-3-nitro-3-methyl-butan-2-ol zur Aminoverbindung der Formel I reduziert.

4. Verfahren nach Anspruch 1 zur Herstellung von 1-[4-(2-n-Propoxyethyl)-phenoxy]-3-amino-3-methyl-butan-2-ol, dadurch gekennzeichnet, daß man 1-[4-(2-n-Propoxyethyl)-phenoxy]-3-nitro-3-methyl-butan-2-ol zur Aminoverbindung der Formel I reduziert.

5. Verfahren zur Herstellung von Arzneimitteln, dadurch gekennzeichnet, daß man eine oder mehrere Verbindungen der allgemeinen Formel I oder deren physiologisch verträgliche Säureadditionssalze mit üblichen galenischen Hilfs- oder Trägermitteln formuliert.

**Claims** (for the Contracting States : BE, CH, DE, FR, GB, IT, LI, LU, NL, SE)

1. Substituted phenoxyalkanolamines and phenoxyalkanol-cycloalkylamines of the general formula I

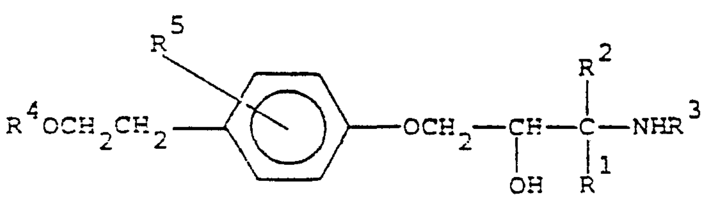

(I)

wherein

$R^1$ and $R^2$ which can be identical or different are an alkyl group having 1 to 4 carbon atoms in each case or

$R^1$ and $R^2$ together are the group $-(CH_2)_n-$,

in which

n is the number 4 or 5,

$R^3$ is hydrogen, an alkyl group or an acyl group each having 1 to 6 carbon atoms,

$R^4$ is an alkyl group having 1 to 4 carbon atoms or the cyclopropylmethyl group and

$R^5$ is hydrogen, halogen or alkyl having 1 to 6 carbon atoms,

and acid addition salts thereof.

2. Phenoxyalkanolamines and phenoxyalkanol-cycloalkylamines according to Claim 1, of the general formula I in which $R^1$ and $R^2$ are each methyl.

3. Phenoxyalkanolamines and phenoxyalkanol-cycloalkylamines according to Claim 1 or 2, of the general formula I in which $R^1$ and $R^2$ are each methyl and $R^4$ is methyl or cyclopropylmethyl.

4. Phenoxyalkanolamines and phenoxyalkanol-cycloalkylamines according to Claim 1, 2 or 3, of the general formula I in which $R^3$ and/or $R^5$ are hydrogen.

5. 1-[4-(2-Methoxyethyl)-phenoxy]-3-amino-3-methyl-butan-2-ol.

6. 1-[4-(2-Cyclopropylmethoxyethyl)-phenoxy]-3-amino-3-methyl-butan-2-ol.

7. Substituted phenoxynitroalkanols and phenoxyhydroxyalkyl-nitrocycloalkanes of the general formula II

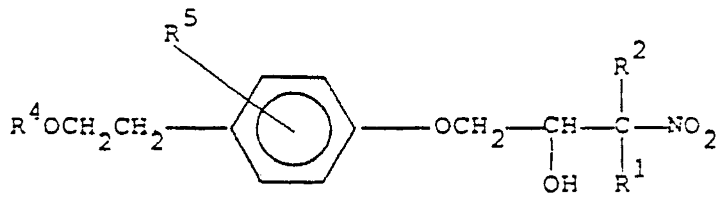

(II)

wherein

$R^1$ and $R^2$ which can be identical or different are an alkyl group having 1 to 4 carbon atoms in each case or

$R^1$ and $R^2$ together are the group $-(CH_2)_n-$,

in which

n is the number 4 or 5,

$R^4$ is an alkyl group having 1 to 4 carbon atoms or the cyclopropylmethyl group and

$R^5$ is hydrogen, halogen or alkyl having 1 to 6 carbon atoms.

8. Process for the preparation of the phenoxyalkanolamines and phenoxyalkanol-cycloalkylamines according to the general formula I, with the above-indicated meaning of the radicals $R^1$ to $R^5$, characterized in that compounds of the general formula II

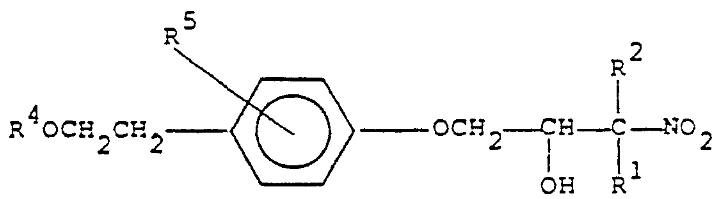

(II)

with the above-indicated meaning of $R^1$, $R^2$, $R^4$ and $R^5$, are reduced and the resulting amino group is, if appropriate, alkylated or acylated.

9. Process for the preparation of the phenoxynitroalkanols and phenoxyhydroxyalkyl-nitrocycloalkanes according to the general formula II, with the above-indicated meaning of the radicals $R^1$, $R^2$, $R^4$ and $R^5$, characterized in that compounds of the general formula III

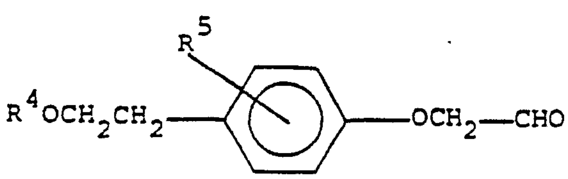

(III)

with the above-indicated meaning of $R^4$ and $R^5$, are reacted with compounds of the general formula IV

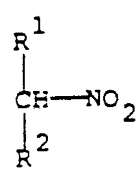

(IV)

with the above-indicated meaning of $R^1$ and $R^2$.

10. Pharmaceutical product, characterized in that it contains one or more of the compounds according to Claim 1 or physiologically acceptable salts thereof and, if appropriate, conventional excipients and/or diluents.

11. Compounds of the general formula I or physiologically acceptable acid addition salts thereof for use as medicaments.

12. Compounds of the general formula I or physiologically acceptable salts thereof for the treatment of glaucoma.

13. 1-[4-(2-n-Propoxyethyl)-phenoxy]-3-amino-3-methyl-butan-2-ol
14. 1-[2-Chloro-4-(2-methoxyethyl)-phenoxy]-3-amino-3-methyl-butan-2-ol

**Claims** (for the Contracting State AT)

1. Process for the preparation of novel substituted phenoxyalkanolamines and phenoxyalkanolcycloalkylamines of the general formula I

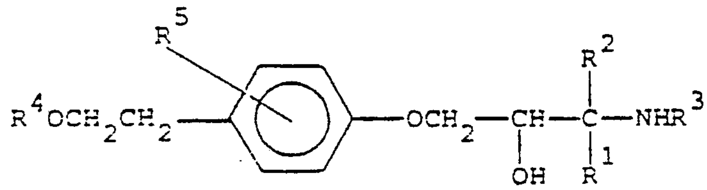

(I)

wherein

$R^1$ and $R^2$ which can be identical or different are an alkyl group having 1 to 4 carbon atoms in each case or

$R^1$ and $R^2$ together are the group $-(CH_2)_n-$,

in which

n is the number 4 or 5,

$R^3$ is hydrogen, an alkyl group or an acyl group each having 1 to 6 carbon atoms,

$R^4$ is an alkyl group having 1 to 4 carbon atoms or the cyclopropylmethyl group and

$R^5$ is hydrogen, halogen or alkyl having 1 to 6 carbon atoms,

and acid addition salts thereof, characterized in that compounds of the general formula II

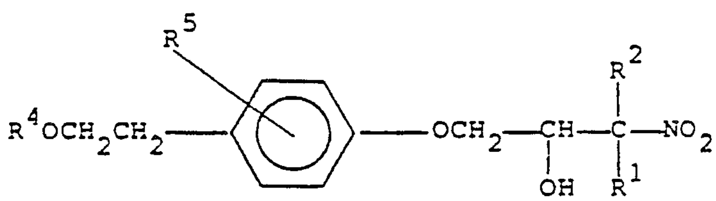

(II)

with the above-indicated meaning of $R^1$, $R^2$, $R^4$ and $R^5$, are reduced and the resulting amino group is, if appropriate, alkylated or acylated.

2. Process according to Claim 1 for the preparation of 1-[4-(2-methoxyethyl)-phenoxy]-3-amino-3-methyl-butan-2-ol, characterized in that 1-[4-(2-methoxyethyl)-phenoxy]-3-nitro-3-methyl-butan-2-ol is reduced to the amino compound of the formula I.

3. Process according to Claim 1 for the preparation of 1-[4-(2-cyclopropylmethoxyethyl)-phenoxy]-3-amino-3-methyl-butan-2-ol, characterized in that 1-[4-(2-cyclopropylmethoxyethyl)-phenoxy]-3-nitro-3-methyl-butan-2-ol is reduced to the amino compound of the formula I.

4. Process according to Claim 1 for the preparation of 1-[4-(2-n-propoxyethyl)-phenoxy]-3-amino-3-methyl-butan-2-ol, characterized in that 1-[4-(2-n-propoxyethyl)-phenoxy]-3-nitro-3-methyl-butan-2-ol is reduced to the amino compound of the formula I.

5. Process for the preparation of medicaments, characterized in that one or more compounds of the general formula I or physiologically acceptable acid addition salts thereof are formulated with conventional galenic auxiliaries or excipients.

**Revendications** (pour les Etats contractants : BE, CH, DE, FR, GB, IT, LI, LU, NL, SE)

1. Phénoxyalcanolamines et phénoxyalcanolcycloalkylamines substituées de formule générale I

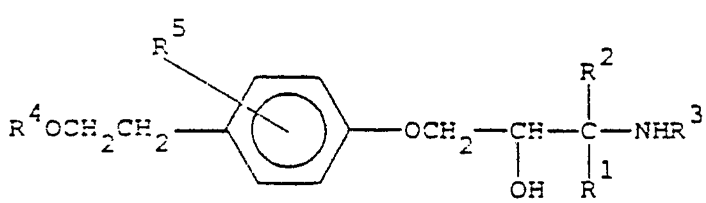

(I)

dans laquelle

$R^1$ et $R^2$, qui peuvent être identiques ou différents, représentent un groupe alkyle ayant chacun de 1 à 4 atomes de carbone ou

$R^1$ et $R^2$ représentent ensemble le groupe $-(CH_2)_n-$,

dans lequel

n est le nombre 4 ou 5,

$R^3$ représente l'hydrogène, un groupe alkyle ou un groupe acyle ayant chacun de 1 à 6 atomes de carbone,

$R^4$ représente un groupe alkyle ayant de 1 à 4 atomes de carbone ou le groupe cyclopropylméthyle et

$R^5$ représente l'hydrogène, un halogène ou un alkyle ayant de 1 à 6 atomes de carbone, ainsi que leurs sels d'addition d'acide.

2. Phénoxyalcanolamines et phénoxyalcanol-cycloalkylamines selon la revendication 1, répondant à la formule générale I, dans laquelle $R^1$ et $R^2$ représentent chacun un méthyle.

3. Phénoxyalcanolamines et phénoxyalcanol-cycloalkylamines selon la revendication 1 ou 2, répondant à la formule générale I, dans laquelle $R^1$ et $R^2$ représentent chacun un méthyle et $R^4$ est un méthyle ou un cyclopropylméthyle.

4. Phénoxyalcanolamines et phénoxyalcanol-cycloalkylamines selon la revendication 1, 2 ou 3, répondant à la formule générale I, dans laquelle $R^3$ et/ou $R^5$ représentent l'hydrogène.

5. [(Méthoxy-2 éthyl)-4 phénoxy]-1 amino-3 méthyl-3 butanol-2.

6. [(Cyclopropylméthoxy-2 éthyl)-4 phénoxy]-1 amino-3 méthyl-3 butanol-2.

7. Phénoxynitroalcanols et phénoxyhydroxyalkylnitrocycloalcanes substitués de formule générale II

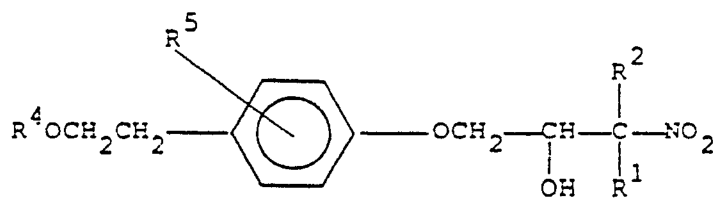

(II)

dans laquelle

$R^1$ et $R^2$ qui peuvent être identiques ou différents, représentent un groupe alkyle ayant chacun de 1 à 4 atomes de carbone ou

$R^1$ et $R^2$ représentent ensemble le groupe $-(CH_2)_n-$

dans lequel

n est le nombre 4 ou 5,

$R^4$ représente un groupe alkyle ayant de 1 à 4 atomes de carbone ou le groupe cyclopropylméthyle et

$R^5$ représente l'hydrogène, un halogène ou un alkyle ayant de 1 à 6 atomes de carbone.

8. Procédé pour la préparation des phénoxyalcanolamines et des phénoxyalcanol-cycloalkylamines répondant à la formule générale I, avec la signification donnée ci-dessus pour les restes $R^1$ à $R^5$, caractérisé en ce que l'on réduit des composés de formule générale II

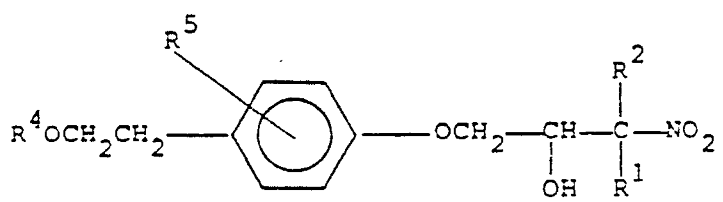

(II)

avec la signification donnée ci-dessus pour $R^1$, $R^2$, $R^4$ et $R^5$, et en ce que l'on alkyle ou acyle, le cas échéant, le groupe amino obtenu.

9. Procédé pour la préparation des phénoxynitroalcanols et des phénoxyhydroxyalkyl-nitrocycloalcanes répondant à la formule générale II, avec la signification donnée ci-dessus pour les restes $R^1$, $R^2$, $R^4$ et $R^5$, caractérisé en ce que l'on fait réagir des composés de formule générale III

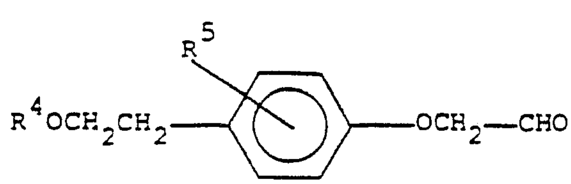

(III)

avec la signification donnée ci-dessus pour $R^4$ et $R^5$, avec des composés de formule générale IV

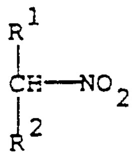

(IV)

avec la signification donnée ci-dessus pour $R^1$ et $R^2$.

10. Préparation pharmaceutique caractérisée en ce qu'elle contient un ou plusieurs composés selon la revendication 1 ou leurs sels physiologiquement acceptables et le cas échéant des excipients usuels et/ou un diluant.

11. Composés de formule générale I ou leurs sels d'addition d'acide physiologiquement acceptables en vue d'une application comme médicament.

12. Composés de formule générale I ou leurs sels physiologiquement acceptables pour le traitement du glaucome.

13. 1-[4-(2-n-Propoxyethyl)-phenoxy]-3-amino-3-methyl-butan-2-ol

14. 1-[2-Chloro-4-(2-methoxyethyl-phenoxy]-3-amino-3-methyl-butan-2-ol

**Revendications** (pour l'Etat contractant AT)

1. Procédé pour la préparation des nouvelles phénoxyalcanolamines et phénoxyalcanol-cycloalkylamine substituées de formule générale I

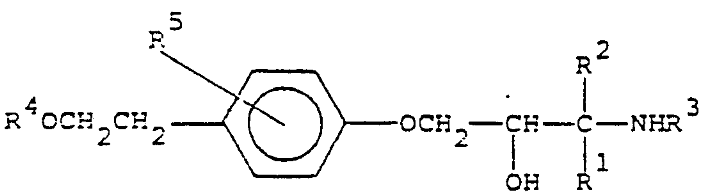

(I)

dans laquelle

$R^1$ et $R^2$, qui peuvent être identiques ou différents, représentent un groupe alkyle ayant chacun de 1 à 4 atomes de carbone ou

$R^1$ et $R^2$ représentent ensemble le groupe $-(CH_2)_n-$, dans lequel

n est le nombre 4 ou 5,

$R^3$ représente l'hydrogène, un groupe alkyle ou un groupe acyle ayant chacun de 1 à 6 atomes de carbone,

$R^4$ représente un groupe alkyle ayant de 1 à 4 atomes de carbone ou le groupe cyclopropylméthyle et

$R^5$ représente l'hydrogène, un halogène ou un alkyle ayant de 1 à 6 atomes de carbone, ainsi que leurs sels d'addition d'acide, caractérisé en ce que l'on réduit des composés de formule générale II

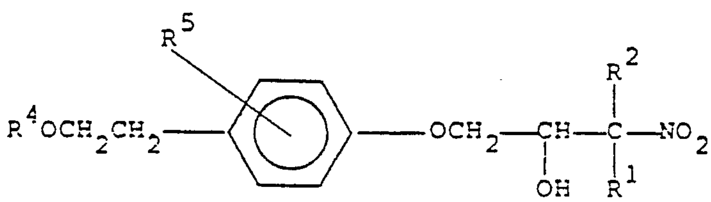

(II)

avec la signification donnée ci-dessus pour $R^1$, $R^2$, $R^4$ et $R^5$, et en ce que l'on alkyle ou acyle, le cas échéant, le groupe amino obtenu.

2. Procédé selon la revendication 1 pour la préparation de [(méthoxy-2 éthyl)-4 phénoxy]-1 amino-3 méthyl-3 butanol-2 caractérisé en ce que l'on réduit le [(méthoxy-2 éthyl)-4 phénoxy]-1 nitro-3 méthyl-3 butanol-2 en le composé amino de formule I.

3. Procédé selon la revendication 1 pour la préparation de [(cyclopropylméthoxy-2 éthyl)-4 phénoxy]-1 amino-3 méthyl-3 butanol-2, caractérisé en ce que l'on réduit le [(cyclopropylméthoxy-2 éthyl)-4 phénoxy]-1 nitro-3 méthyl-3 butanol-2 en le composé amino de formule I.

4. Procédé selon la revendication 1 pour la préparation de [(n-propoxy-2 éthyl)-4 phénoxy]-1 amino-3 méthyl-3 butanol-2, caractérisé en ce que l'on réduit le [(n-propoxy-2 éthyl)-4 phénoxy]-1 nitro-3 méthyl-3 butanol-2 en le composé amino de formule I.

5. Procédé de préparation de médicaments, caractérisé en ce que l'on formule un ou plusieurs composés de formule générale I ou leurs sels d'addition d'acide physiologiquement acceptables avec des adjuvants ou des excipients galéniques usuels.